(19) Europäisches Patentamt
European Patent Office
Office européen des brevets

(11) EP 1 196 418 B1

(12) **FASCICULE DE BREVET EUROPEEN**

(45) Date de publication et mention de la délivrance du brevet:
**16.06.2004 Bulletin 2004/25**

(21) Numéro de dépôt: **00949588.8**

(22) Date de dépôt: **29.06.2000**

(51) Int Cl.[7]: **C07D 487/06**, C07D 519/00 // (A61K31/5517, A61P29:00, 11:00), (C07D487/06, 243:00), C07D209:00,(C07D519/00, 487:00), C07D471:00

(86) Numéro de dépôt international:
**PCT/FR2000/001839**

(87) Numéro de publication internationale:
**WO 2001/002403 (11.01.2001 Gazette 2001/02)**

# (54) PROCEDE DE PREPARATION DE 1,4]DIAZEPINO 6,7,1- i hi /i ]INDOL-4-ONES SUBSTITUEES

VERFAHREN ZUR HERSTELLUNG VON SUBSTITUIERTEM [1,4]DIAZEPINO[6,7,1-HI]INDOL-4-ONE

METHOD FOR PREPARING SUBSTITUTED 1,4]DIAZEPINO 6,7,1- i hi /i ]INDOL-4-ONES

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **02.07.1999 FR 9908537**

(43) Date de publication de la demande:
**17.04.2002 Bulletin 2002/16**

(73) Titulaire: **Warner-Lambert Company LLC**
**Morris Plains, New Jersey 07950 (US)**

(72) Inventeur: **BERNARDELLI, Patrick**
**F-92260 Fontenay-aux-Roses (FR)**

(74) Mandataire: **Hirsch, Denise et al**
**PFIZER PGRD**
**European Pharma Patent Dept.**
**23/25 Avenue du Docteur Lannelongue**
**75668 Paris Cedex 14 (FR)**

(56) Documents cités:
**WO-A-98/49169**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 196 418 B1

Printed by Jouve, 75001 PARIS (FR)

## Description

DOMAINE DE L'INVENTION

La présente invention est relative à une nouvelle méthode de préparation de [1,4]diazépino[6,7,1-*hi*]indol-4-ones chirales, utiles pour la préparation de médicaments permettant de traiter des affections relevant d'une thérapie par un inhibiteur de phosphodiestérases 4 (PDE4). Ces médicaments sont utiles notamment comme anti-inflammatoires, anti-allergiques, bronchodilatateurs, ou anti-asthmatiques.

La demande de brevet internationale publiée sous le n° WO 98/49169, dont le contenu est incorporé à la présente demande par référence, décrit des diazépino-indolones, qui sont des composés actifs comme inhibiteurs d'enzymes PDE4, de formule

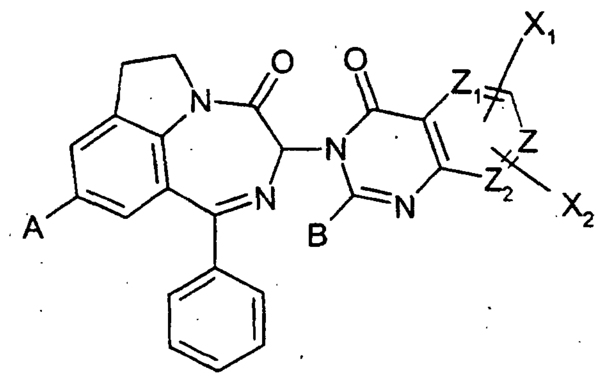

dans laquelle:

- A est hydrogène, alkyle inférieur, alcoxy inférieur, nitro ou amino ;
- B est hydrogène, ou alkyle inférieur éventuellement fonctionnalisé ;
- $X_1$ et $X_2$ , semblables ou différents, peuvent être hydrogène, halogène, alkyle inférieur, alcoxy inférieur, ou encore $-CH_2OH$ ou $-CO_2H$ éventuellement substitués.
- Z est CH, alors $Z_1$ et $Z_2$ sont tous deux CH ou N ; ou Z est N, alors $Z_1$ et $Z_2$ sont CH.

Dans cette demande WO 98/49169, on préfère les diazépino-indolones de formule (I) pour lesquelles le carbone 3 du noyau [1,4]diazépino[6,7,1-*hi*]-indol-4-one possède la configuration S.

Ces composés étaient obtenus dans la demande de brevet WO 98/49169 comme racémiques, et ne pouvaient être séparés que par chromatographie sur phase chirale, ou bien par la formation de sels avec une amine énantiomériquement pure. Le procédé de synthèse décrit dans la demande de brevet WO 98/49169 peut présenter des inconvénients y compris à l'occasion des rendements faibles, et la nécessité d'une étape de résolution.

SOMMAIRE DE L'INVENTION

Or on a maintenant trouvé un procédé amélioré, objet de la présente invention, efficace et économique, pour la préparation de ces mêmes produits directement sous forme d'énantiomères purs. Par conséquent, le présent procédé évite les inconvénients des procédés connus, et il peut être transposé sur une plus grande échelle.

L'invention concerne un procédé amélioré de préparation de [1,4]diazépino[6,7,1-*hi*]indol-4-ones substituées chirales de formule (1)

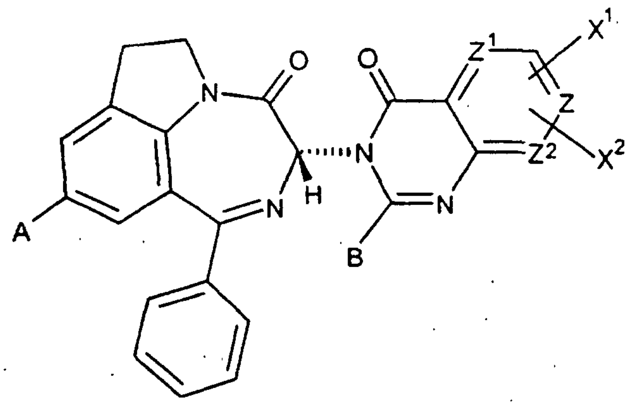

(I)

dans laquelle notamment :

- A est hydrogène, alkyle inférieur, alcoxy inférieur, nitro ou amino ;
- B est hydrogène, ou alkyle inférieur ;
- Z est CH, alors $Z^1$ et $Z^2$ sont ensemble CH ou N ; ou Z est N, alors $Z^1$ et $Z^2$ sont CH ;
- $X^1$ et $X^2$ , semblables ou différents, peuvent être hydrogène, halogène, allyle inférieur, alcoxy inférieur, ou encore $-CH_2OH$ ou $-CO_2H$ éventuellement substitués,

DESCRIPTION DETAILLEE DE L'INVENTION

L'invention vise un procédé de préparation des énantiomères des diazépino-indolones (I) de formule

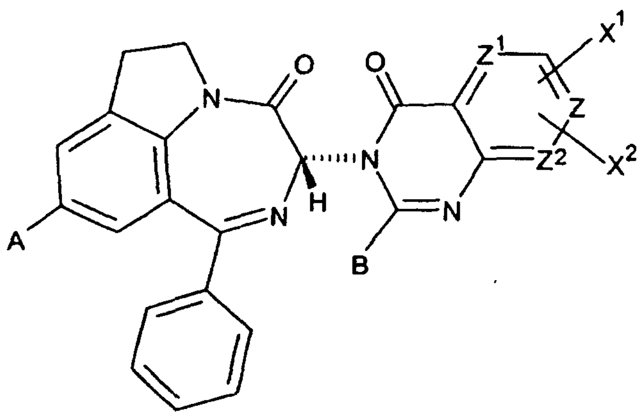

(I)

dans laquelle :

- A est hydrogène, $C_1$-$C_4$-alkyle, hydroxy, $C_1$-$C_4$- alcoxy, nitro, cyano, ou $NR^1R^2$ ; $R^1$ et $R^2$ sont indépendamment hydrogène, $C_1$-$C_4$ alkyle, ou forment ensemble avec l'atome d'azote auquel ils sont liés un cycle ayant 4 ou 5 atomes de carbone ;
- B est hydrogène ou $C_1$-$C_4$ alkyle;
- Z est CH, alors $Z^1$ et $Z^2$ sont ensemble CH ou N ; ou Z est N, alors $Z^1$ et $Z^2$ sont CH;
- $X^1$ et $X^2$ sont indépendamment hydrogène, $C_1$-$C_4$ alkyle, $-(CH_2)_j-OR^3$, halogène, cyano, - O-$C_1$-$C_6$ alkyle, $-C(=O)R^4$, $-C(=O)OR^5$, $-C(=O)NR^6R^7$, ou

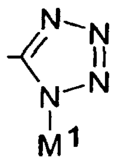 , ou 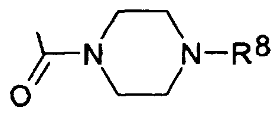 ;

- $R^3$, $R^4$, et $R^5$ sont indépendamment hydrogène, $C_1$-$C_6$ alkyle, benzyle,phénéthyle, ou - $Q^1$-$Q^2$;
- $R^6$ est hydrogène, ou $C_1$-$C_4$ alkyle;
- $R^7$ est hydrogène, $C_1$-$C_4$ alkyle, $-CHR^A-C(=O)OM^2$, ou $-Q^3-Q^4$ ;
- $R^8$ est hydrogène, $C_1$-$C_4$ alkyle, ou $-Q^5-Q^6$ ;
- $R^A$ est un résidu d'α-amino-acide naturel, l'atome de carbone auquel il est lié pouvant avoir soit la configuration S, soit la configuration R ;
- $Q^1$ est $-(CH_2)_k-(CHOH)_m-(CH_2)_p-$ ;
- $Q^2$ est hydroxy, -O- $C_1$-$C_6$ alkyle, -OC(=O)- $C_1$-$C_6$ alkyle, ou 4-morpholinyle ;
- $Q^3$ et $Q^5$ sont indépendamment une liaison, $-CH_2$ -, $-(CH_2)_2$ -, ou $-(CH_2)_3$ - ;
- $Q^4$ est $-NM^3M^4$, ou 4-morpholinyle ;
- $Q^6$ est $-M^5$ ou $-OM^6$;
- $M^1$, $M^2$, $M^3$, $M^4$, $M^5$, et $M^6$ sont indépendamment hydrogène ou $C_1$-$C_4$ alkyle ;
- j est 1,2, ou3 ; k est 1,2,ou 3 ;
- m est 0, 1, 2, 3, ou 4 ; p est 0, 1, 2, ou 3, sous réserve que si m > 0, alors p > 0; leurs sels et solvates, ,

qui comprend la cyclisation intramoléculaire à une température qui ne dépasse pas 40 °C, d'un produit de formule

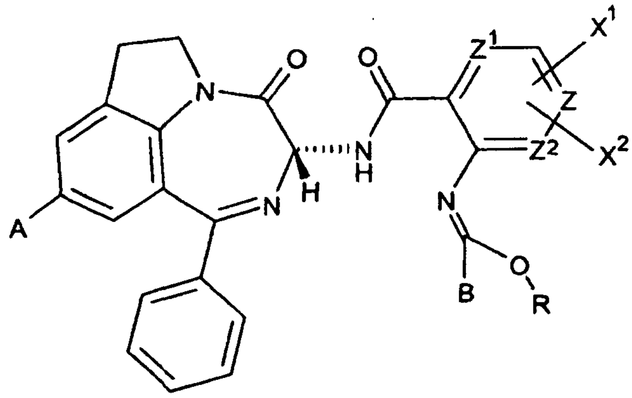

,

dans laquelle, A, Z, $Z^1$, $Z^2$, B, $X^1$ et $X^2$ ont la signification définie ci-dessus, et R est $C_1$-$C_4$ alkyle, en présence d'un catalyseur acide de Lewis faible qui est le scandium trifluorométhanesulfonate ; l'aluminium trifluorométhanesulfonate ; les trifluorométhanesulfonates de lanthanide, de silicium, de magnésium, d'étain (II), de cuivre (II), de zinc ou d'argent ; le diméthoxy- dicyclopentadiényl-titane (IV); le bis (trifluorométhanesulfonate)dicyclopentadiényl-titane (IV) ; l'acétylacétonate de fer (III), d'aluminium
ou de zinc ; le diacétate de zinc ; le diméthoxy magnésium ; le triisopropoxy aluminium ; le tétrabutoxy titane (IV) ; le tétraisopropoxy titane (IV) ; le triméthyl bore ; le triéthyl aluminium ; le diéthyl zinc ; le triisobutyl aluminium ; le tétrabutyl étain (IV) ; le triphényl bore ; le triphényl antimoine ; ou les halogénures de, zinc, d'étain (II), d'antimoine, de titane, de scandium, d'indium, de gallium, ou de mercure (II).

Dans ce qui précède et dans ce qui suit :

- par 'halogène' on entend le fluor, le chlore, le brome ou l'iode ;
- par 'alkyle inférieur' ou par '$C_1$-$C_4$ alkyle', on entend un radical linéaire ou ramifié, comportant de un à quatre atomes de carbone, ou encore le radical cyclopropylméthyle ;
- par alcoxy inférieur, on entend un radical de formule -O-Alk, où Alk est alkyle inférieur ;
- par '$C_1$-$C_6$ alkyle', on entend un radical linéaire ou ramifié, comportant de un à six atomes de carbone, ou encore le radical cyclopropylméthyle.

Le procédé de la présente invention permet d'obtenir les [1,4]diazépino[6,7,1-*hi*]indol-4-ones chirales (I) directement, sous forme d'énantiomères purs, à partir de 3-amino benzodiazépines (II) optiquement actives, en une étape présentée au Schéma 1.

**Schéma 1 :**

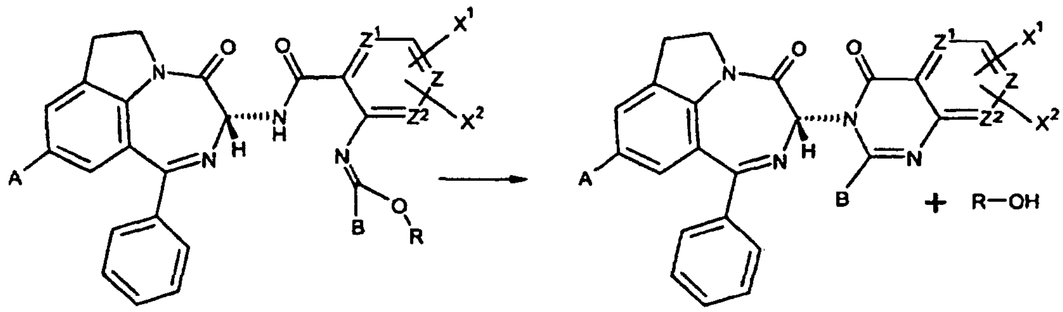

(II) (I)

où A, Z, $Z^1$, $Z^2$, B, $X^1$, et $X^2$ ont les désignations définies ci-dessus pour (I) ;
R est un radical alkyle inférieure, de préférence, le radical méthyle.

Lors de cette réaction, un intermédiaire (II) est cyclisé, de préférence à température ordinaire ou en dessous de 0 °C en présence d'un acide de Lewis, tel que le trifluorométhanesulfonate de scandium, pour donner le produit (I).

L'intermédiaire (II) est dissous dans un solvant et on ajoute une quantité catalytique d'acide de Lewis, de préférence du trifluorométhanesulfonate de scandium, à température ordinaire. On obtient le produit (I), dont la pureté optique est vérifiée par HPLC analytique.

Plus généralement, les acides de Lewis utiles dans le procédé de la présente invention sont décrits notamment dans les publications suivantes : i) *Advanced Organic Chemistry,* Third Edition, par Jerry March (John Wiley & Sons, New York, 1985); ii) « Friedel-Crafts Reactions » Olah, G. et Meidar, D. *; Kirk-Othmer Encyclopedia of Chemical Technology,* Third Edition, 11 : 269-300 (John Wiley & Sons, New York, 1978), iii) « Quantitative Aspects of Lewis Acidity » Satchell, D.P.N. et Satchell, R.S. ; *; Quarterly Reviews* (The Chemical Society London), 1971, 25 : 171-199, iv) « Lanthanide triflates as unique Lewis acids » Xie, W.; Jin, Y.; Wang, *P.G. ;CHEMTECH,* 1999, **29,** 23-29, et v) Marshman, R.W. « Rare earth triflates in organic synthesis *»Aldrichimica Acta,* 1995, **28** 77-84.

Les acides de Lewis forts (tels que le chlorure d'aluminium, le chlorure ferrique , et équivalents) n'apparaissent pas dans la présente invention des catalyseurs aussi efficaces que les acides de Lewis faibles.

Un exemple spécifique d'un acide de Lewis faible utile dans la présente invention est le scandium trifluorométhanesulfonate,

La réaction de cyclisation est conduite dans un solvant inerte, c'est-à-dire un solvant ou mélange de solvants ne réagissant pas avec les réactifs ou les produits de la réaction, et ne réagissant pas de manière défavorable avec le catalyseur acide de Lewis. Le solvant est aprotique, et de préférence peu polaire. Des solvants représentatifs sont : des hydrocarbures aromatiques comme le benzène, le toluène, le nitrobenzène, ou le chlorobenzène; des hydrocarbures aliphatiques comme le pentane, l'hexane ou l'heptane ; des dialkyl-éthers comme l'éther éthylique ou isopropylique; des hydrocarbures chlorés comme le dichlorométhane, le trichlorométhane, le tétrachlorométhane ou le dichloroéthytène ; des 1,1,1-triméthoxy-alcanes comme le triméthyl orthoacétate ; des éthers cycliques comme le tétrahydrofurane, ou le dioxanne ; et leurs mélanges. Il n'est pas nécessaire que les réactifs ou le catalyseur soient complètement solubilisés dans le solvant utilisé.

La quantité d'acide de Lewis utilisée représente en général de 1 à 10% équivalent en mole par rapport au produit de départ (II). Dans cette réaction de cyclisation, la quantité minimale d'acide de Lewis relativement au produit de formule (II) dépend de l'activité de l'acide de Lewis considéré, de la température de la réaction et de sa durée maximale allouée ; sa détermination se fait par des expérimentations de routine. Avantageusement, en fin de réaction l'acide de Lewis est facilement recyclable.

Le catalyseur acide de Lewis est de préférence soluble dans le solvant utilisé.

Facultativement, un agent déshydratant, comme le sulfate de magnésium anhydre ou un tamis moléculaire, peut être introduit dans le milieu réactionnel de la cyclisation.

La réaction de cyclisation est conduite à une température comprise entre environ 0°C et 40°C, et de préférence entre environ 25°C et 40°C. Il convient de ne pas dépasser 40°C pour ne pas épimériser le centré stéréogénique.

La durée de la réaction est généralement comprise entre 6 et 48 h. Son évolution peut être suivie par CCM ou HPLC analytique ; ainsi la réaction est stoppée après disparition du produit de départ.

Lorsque dans le produit final A est un groupe amine primaire, le procédé comporte une étape additionnelle où un produit (**I**) dans lequel A est $-NO_2$ est réduit en un produit (I) dans lequel A est $-NH_2$ par une réduction chimique ou catalytique qui respecte le carbone asymétrique. Cette étape consiste à réduire de façon spécifique un composé (I) dans lequel A est nitro, ce que l'on réalise par des systèmes réducteurs appropriés : ce sont, entré autres, le zinc ou le chlorure de titane en milieu acide, ou bien le chlorure d'étain en milieu éthanolique ; cette étape est représentée dans la partie expérimentale ci-après par la synthèse des produits **7**, **13**, et **18**.

Ces réductions peuvent se faire à froid, mais la réduction préférée est conduite dans le méthanol, avec du ruthénium sur charbon comme catalyseur, à une température n'excédant pas 80 °C, et sous une pression d'hydrogène de 400 à 800 kPa.

Le temps d'hydrogénation doit être inférieur à 2 h, et de préférence 1,5 h.

L'intermédiaire (II) peut être préparé selon le procédé présenté au Schéma 2, où A, Z, $Z^1$, $Z^2$, $X^1$ et $X^2$ ont les désignations définies pour (I), et PG est un groupement protecteur.

Schéma 2

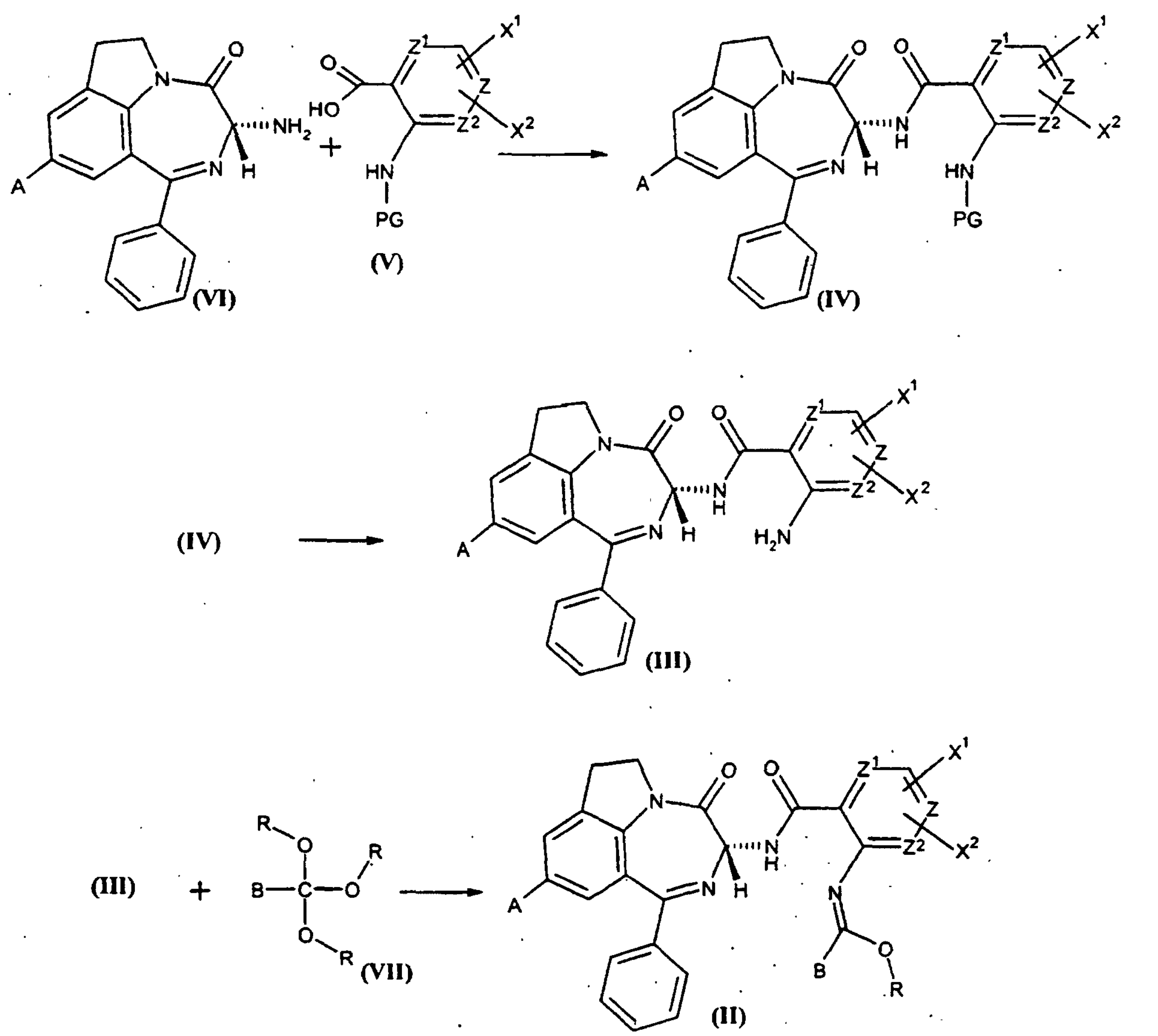

(VI) (V) (IV)

(IV) (III)

(III) (VII) (II)

La première étape consiste en la condensation d'une amino benzodiazépine (VI) optiquement active avec un acide anthranilique (V) convenablement substitué et protégé sur la fonction amine, pour obtenir un intermédiaire (IV).

L'intermédiaire (IV) est ensuite déprotégé pour donner l'intermédiaire (III) avec une fonction amine libre.

L'intermédiaire (III), mis à réagir avec un ortho-ester (ou 1,1,1-trialkoxy-alcane) approprié (VII) dans des conditions douces, pour ne pas épimériser le carbone asymétrique de la benzodiazépine, donne un intermédiaire (II).

Dans certains cas, on peut condenser directement un acide anthranilique (V') non protégé' sur la fonction amine avec l'amino-benzodiazépine (VI), ce qui fournit directement le composé intermédiaire (III) (procédé B).

De façon plus précise, le procédé A, tel que présenté au Schéma 2, comprend la préparation des intermédiaires (IV), par réaction d'un intermédiaire aminé (VI) avec un intermédiaire (V) préparé à partir d'un 2-acide anthranilique. Il consiste, avec un 2-acide anthranilique protégé (V), à réaliser dans une première étape la N-acylation de l'amine (VI). L'opération est réalisée dans un solvant organique anhydre comme un hydrocarbure chloré tel que le dichlorométhane ou le trichlorométhane, un éther-oxyde linéaire ou cyclique comme le diméthoxy-1,2-éthane, le tétrahydrofurane ou le dioxanne, un solvant polaire aprotique tel que la pyridine, le diméthylsulfoxyde, le N,N-diméthylformamide (DMF) ou tout autre solvant convenable et leurs mélanges. Avantageusement on réalise la réaction en présence d'un agent de condensation et éventuellement d'une base organique. Ainsi on utilise comme agent de condensation :

- une association tétrafluoroborate de O-[(éthoxycarbonyl)-cyanométhylamino]-N,N,N',N'-tétraméthyluronium / N. N-diisopropyl-éthylamine, ou
- une association chloroformate d'isobutyle /N-méthyl-morpholine, ou
- de préférence, une association d'hydroxybenzotriazole (HOBT) et d'un carbodiimide, comme le N,N'-dicyclohexylcarbodiimide (DCC), qui est le réactif préféré, le N,N'-disopropylcarbodiimide ou le carbonyl-diimidazole, dans un solvant neutre anhydre tel que le dichlorométhane ou le DMF à 0 °C.

Dans cette première étape du procédé A, on engage le *t*-butoxycarbonylamino-acide (V) sous forme isolée.
La deuxième étape consiste à déprotégér la fonction amine de l'intermédiaire (IV) pour obtenir l'amine primaire (III). Dans ce but, l'intermédiaire (IV) peut être dissous dans un acide tel que l'acide trifluoroacétique, et c'est le procédé préféré, et laissé réagir pendant un temps qui dépend de la nature du groupe protecteur employé. Dans le cas du groupe protecteur PG préféré, qui est le groupe *t*-butoxycarbonyle, ci-après «*t*-BOC », les produits sont laissés en contact pendant une demi-heure, et le mélange est évaporé. Le produit est repris dans du dichlorométhane et neutralisé par une solution aqueuse de $NaHCO_3$ à 5%. On obtient l'intermédiaire (III).
Dans la partie expérimentale qui illustre l'invention, les Exemples 1, 2, 4, et 5 sont représentatifs du procédé A.
L'intermédiaire (III) peut aussi, avec un rendement éventuellement moindre, être obtenu par condensation directe d'un acide anthranilique non protégé sur la fonction amine (V') avec l'amine énantiomère (VI) de départ, par le procédé B.
Dans ce cas l'agent de condensation peut être aussi l'association DCC/HOBT. Pour cette étape on peut employer d'autres acides tels que l'acide formique, l'acide dichloroacétique, ou tout autre acide organique ou minéral mélangé ou non à un solvant dans des proportions variables, à une concentration et à une température telles qu'il n'y ait pas hydrolyse du noyau benzodiazépine.
Dans la partie expérimentale, l'Exemple 3 est représentatif du procédé B.

Procédé B :

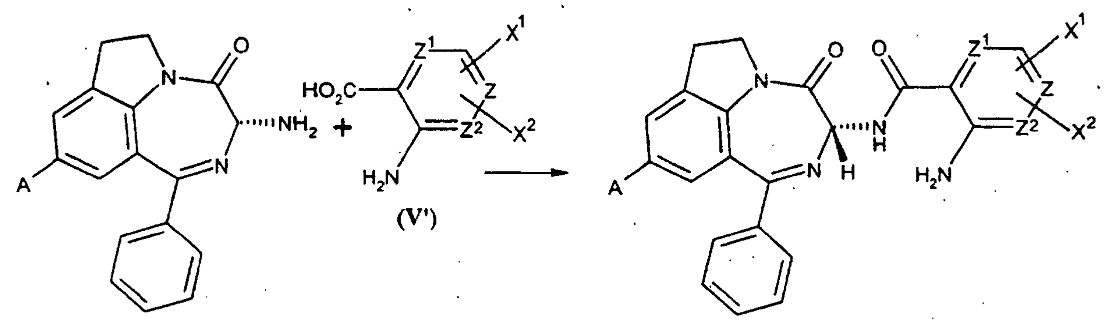

(VI) (V') (III)

A partir de l'intermédiaire (III), les étapes suivantes sont communes aux deux procédés.
Dans la troisième étape, on agite une solution de l'intermédiaire (III) avec un ortho-ester (VII), de préférence un ortho-ester de méthyle, pour obtenir l'intermédiaire (II) qui n'est pas purifié mais employé tel quel dans l'étape suivante. Au cours de cette troisième étape, il convient de rester à basse température, et surtout de ne pas dépasser 40 °C, faute de quoi (III) et (II) s'épimériseraient pour donner un mélange racémique. La durée de la condensation peut atteindre 24 h, mais elle est parfois beaucoup plus rapide. On peut, par distillation sous vide, éliminer le méthanol qui se forme pendant la réaction ; la réaction est ainsi complète.

Les acides anthraniliques substitués protégés (V) sont décrits dans la littérature ; sinon ils sont préparés de façon analogue à la préparation de l'acide 2-*t*-butoxycarbonylamino-benzoïque **2,** décrite dans la partie expérimentale ci-après.

PARTIE EXPERIMENTALE

Les exemples suivants illustrent, sans pour autant la limiter, la mise en oeuvre du procédé selon l'invention, et les produits obtenus. Le procédé décrit dans la présente demande illustre la préparation d'un produit de formule (I), qui est un énantiomère S. La pureté, l'identité et les caractéristiques physico-chimiques des produits et des intermédiaires essentiels préparés sont déterminées; ainsi :

- la pureté est vérifiée par chromatographies sur couches minces (C.C.M.) de gel de silice (Merck 60 - F254) et le Rf observé est rapporté pour le solvant d'élution utilisé qui est, le plus fréquemment, identique à celui utilisé pour l'analyse chromatagraphique des composés. Ces solvants sont identifiés par les sigles suivants :

  D/M1 : dichlorométhane méthanol, ,99/1 (v/v),
  D/M2 : dichlorométhane / méthanol, ,98/2 (v/v),
  D/M5 : dichlorométhane / méthanol , 95 / 5 (v/v),
  D/MN5 : dichlorométhane / méthanol 10% ammoniacal, 95/5 (v/v),
  D/MN20 : dichlorométhane / méthanol 10% ammoniacal, 80 / 20 (v/v),

- l'identité des produits obtenus avec les structures proposées est vérifiée par leur spectre de résonance magnétique nucléaire du proton et par leur spectrographie infrarouge.

Les spectres R.M.N $^1$H sont réalisés à 400 MHz sur un appareil de marque Brüker, les composés étant dissous dans le deutérochloroforme avec le tétraméthylsilane comme référence interne. La nature des signaux, leurs déplacements chimiques en ppm, le nombre de protons qu'ils représentent sont notés.
Les spectres infrarouge sont enregistrés en pastille de bromure de potassium sur un spectromètre Shimadzu IR-435.
La pureté optique des différents énantiomères est vérifiée sur un système de chromatographie liquide sous haute pression analytique de type Merck, par injection sur une colonne chirale Pirkle D-phényl-glycine 250 x 4,6 mm, particules de 5 μm, thermostatée à 35 °C, éluant: hexane/éthanol 50/50 (v/v), débit 1ml/min. Le résultat calculé sous la forme d'excès énantiomérique (ee) est donné par la formule:

$$ee = \frac{\text{masse d'énantiomère (S) - masse d'énantiomère (R)}}{\text{masse d'énantiomère (S) + masse d'énantiomère (R)}}$$

- les caractéristiques physico-chimiques relevées dans la mesure d'une disponibilité suffisante de produit sont le point de fusion non corrigé, déterminé par la méthode du tube capillaire, et le pouvoir rotatoire, déterminé à la température ambiante voisine de 20 °C sur un appareil Polartronic en cuve de 10 cm de long.

En ce qui concerne la description expérimentale :

- par concentration ou élimination des solvants, on entend, éventuellement après leur dessication sur un agent déshydratant approprié comme $Na_2SO_4$ ou $MgSO_4$, une distillation sous un vide de 25 à 50 mm Hg (3,3 à 6,7 kPa) et avec un chauffage modéré sur bain-marie (à une température inférieure à 30 °C);
- par chromatographie rapide sur colonne de silice, on entend la mise en oeuvre d'une méthode adaptée de celle de Still *et al.* (1978) J.Org.Chem. 43: 2923, la pureté des fractions d'élution étant vérifiée avant leur regroupement et leur évaporation dans les conditions définies ci-dessus.

EXEMPLE 1

(3S) 3-(2-méthyl-4-oxo-4*H*-quinazolin-3-yl)-9-amino-1 -phényl-6,7-dihydro-3*H*-[1,4]diazépino[6,7,1-*hi*]indol-4-one. **(7)**

[(I) ; A = $NH_2$, Z = CH, $Z^1$ = CH, $Z^2$ = CH, B = $CH_3$ , $X^1$ = H, $X^2$ = H] (procédé A)

1°) Synthèse de l'intermédiaire (II)

Intermédiaire **1** : (3R) 3-amino-9-nitro-1-phényl-6,7-dihydro-3*H*-[1,4]diazépino[6,7,1-hi]indol-4-one. [(VI); A = $NO_2$].

Dans un réacteur de 100 ml, on introduit 51,0 ml d'acide sulfurique concentré (d = 1,83) et sous agitation on ajoute 16,0 g (57,7 mmol) de (3R) 3-amino-1-phényl-6,7-dihydro-3*H*-[1,4]diazépino[6,7,1-*hi*]indol-4-one, préparé tel que décrit à l'intermédiaire 1.b du brevet

FR 94 12282. Durant l'introduction exothermique, la température atteint 70 °C ; la solution marron obtenue est refroidie à 5-10 °C. On introduit alors rapidement 6,93 g (68,5 mmol) de nitrate de potassium pur dissous dans 17,0 ml d'acide sulfurique (d = 1,83). La température progresse à 40 °C puis on maintient 40 min sous agitation à 20 °C. La solution brune est précipitée dans 600 ml d'un mélange eau et glace. Le mélange est alcalinisé avec une solution d'ammoniaque concentrée puis extrait par 3 fois 150 ml de dichlorométhane.

Les phases organiques sont lavées à l'eau, déshydratées, les solvants sont ensuite éliminés par distillation. On obtient un résidu meringué marron clair (17,5 g) qui est purifié par chromatographie rapide sur silice. L'élution par du dichlorométhane progressivement enrichi en méthanol permet d'obtenir 12,0 g de produit purifié **1**.
Rdt = 75 % F = 177-178°C
$[\alpha]_D$ (c = 0,4, $CH_2Cl_2$)= + 66,8 °C.

Intermédiaire **2** : acide 2-*t*-butoxycarbonylamino-benzoïque.

$$[(V) ; Z = CH, Z^1 = CH, Z^2 = CH, X^1 = H, X^2 = H]$$

Dans un réacteur protégé de l'humidité on dissout sous agitation 22 g (160 mmol) d'acide anthranilique dans 300 ml de méthanol contenant 10% de triéthylamine. On ajoute 38,68 g de di-*t*-butyl-dicarbonate et chauffe au reflux pendant 4 h. On évapore, reprend par de l'acétate d'éthyle, lave par une solution normale de $KHSO_4$, lave par de l'eau: On évapore, reprend par du dichlorométhane, filtre l'insoluble : 20 g du produit attendu (V). On chromatographie sur silice le résidu dans le dichlorométhane enrichi en acétate d'éthyle.

On obtient encore 9 g du produit **2** attendu. Rdt global : 76%.

Intermédiaire **3** : acide [2-((3R) 9-nitro-4-oxo-1-phényl-3,4,6,7-tétrahydro-[1,4] diazépino[6,7,1-*hi*]indol-3-ylcarbamoyl)-phényl]-carbamique *t*-butyl ester.

$$[(IV) ; A = NO_2 , Z=CH, Z^1 = CH, Z^2 = CH, X^1 = H, X^2 = H, PG = t\text{-BOC}]$$

Dans un réacteur protégé de l'humidité on dissout sous agitation 7 g (32 mmol) de **2** dans 250 ml de dichlorométhane sec. Le mélange est refroidi à 0 °C et on ajoute 10 g (32 mmol) de **1** dans 30 ml de dichlorométhane. On ajoute 4,32 g (32 mmol) d'hydroxybenzotriazole (HOBT) et 6,6 g (32 mmol) de dicyclohexylcarbodiimide (DCC).

Après 4 h sous agitation à 0 °C, l'insoluble est filtré et le filtrat extrait successivement par une solution $HKSO_4$ 0,1 N, une solution saturée en $NaHCO_3$ et enfin par de l'eau. Le solvant est évaporé sous vide à 0 °C et le résidu purifié par chromatographie rapide sur colonne de silice en éluant par le solvant dichlorométhane /acétate d'éthyle à 5%. On obtient 10 g d'un solide blanc cristallisé. Rdt = 60 %.
C.C.M. (D/M1) : Rf = 0,43.
H.P.L.C. (colonne Pirkle / D-phényl-glycine, éluant hexane/éthanol 50/50, T=35°C, débit : 1 ml/min, UV : 254 nm) : ee = 93.2 %
R.M.N. $^1H$ $\delta$ (ppm) : 1,5 (s,9H); 3,2-3,6(m,2H); 4,05-4,25(m, 2H); 4,75 (m, 1H); 5,65 (d, 1H); 7,08 (t, 1H); 7,4-7,6 (m, 5H); 7,80 (d, 1H); 7,88(d, 1H); 8,22 (d, 2H); 8,3 (s,1H); 8,40-8,45 (d, 1H); 10,1 (s,1H).

Intermédiaire **4** : 2-amino-*N*-((3R) 9-nitro-4-oxo-1-phényl-3,4,6,7-tétrahydro-[1,4] diazépino[6,7,1-*hi*]indol-3-yl)-benzamide.

$$[ (III) ; A = NO_2, Z=CH, Z^1 = CH, Z^2 = CH, X^1 = H, X^2 = H]$$

Dans un réacteur de 50 ml protégé de l'humidité et sous atmosphère d'azote, on introduit 10 g (19 mmol) de **3** dans 500 ml de dichlorométhane, puis à 0 °C, 75 g (665 mmol) d'acide trifluoroacétique. On agite 1 h à 0 °C et on évapore à 0 °C sous vide. On reprend par de l'acétate d'éthyle, on lave par une solution de $NaHCO_3$ puis par une solution de NaCl, et évapore à 0 °C. Brut : 8,4 g.

Le produit est employé brut dans l'étape suivante.
C.C.M. (D/M1) : Rf= 0,29.
ee : 95,9 %.
R.M.N. $^1H$ $\delta$ (ppm) : 3,25 (s,2H, échangeables); 3,1-3,5 (m,2H); 3,9-4,12 (m,1H) 4,4-4,6 (m,1H); 5,12 (d,1H); 6,35 (s, 1H,échangeable); 6,45-6,6 (t,1H); 6,65 (d,1H); 7,12 (t,1H); 7,35-7,6 (m,4H); 7,6-7,9 (m,1H); 7,9-8,1 (m,1H); 8,38(s, 1H); 9,25(m,1H).

Intermédiaire **5** : acide *N*-[2-((3R) 9-nitro-4-oxo-1-phényl-3,4,6,7-tétrahydro-[1,4] diazépino[6,7,1-*hi*]indol-3-ylcarbamoyl)-phényl]-acétimidique méthyl ester.

$$[(II) ; A = NO_2 , Z=CH, Z^1 = CH, Z^2 = CH, B = CH_3, X^1 =H, X^2 = H, R = CH_3]$$

Dans un réacteur protégé de l'humidité et sous atmosphère d'azote on introduit 500 ml de triméthyl orthoacétate et 8,4 g de **4.** On place le réacteur agité sous un vide de 0,1 kPa et on chauffe à 40 °C pendant 6 h. On évapore sous vide, la RMN confirme l'existence du nouveau produit **5** : 10 g de brut réactionnel.
HPLC : ee > 95 %.
CCM (D/M2) : Rf = 0,87.
R.M.N. $^1H$ $\delta$ (ppm) : 1,95 (s,3H); 3,1-3,6 (m,2H); 4,0 (s,3H); 3,95-4,22 (m,1H); 4,65-4,8 (m,1H); 5,7 (d,1H); 6,85 (d, 1H); 7,15-7,30 (m,1H); 7,35-7,55 (m,6H); 8,15-8,25(m,2H); 8,3 (s,large,1H); 9,65 (d,1H).

2°) (3S) 3-(2-méthyl-4-oxo-4*H*-quinazolin-3-yl)-9-amino-1 -phényl-6,7-dihydro-3*H*- [1,4]diazépino[6,7,1-*hi*]indol-4-one. (7)

Produit **6**: (3S) 3-(2-méthyl-4oxo-4H-quinazolin-3yl)-9-nitro-1-phényl-6,7-dihydro-3*H*[1,4]diazépino[6,7,1-*hi*]indol-4-one.

$$[(I) ; A = NO_2 , Z=CH, Z^1 = CH, Z^2 = CH, X^1 = H, X^2 = H]$$

Dans un réacteur protégé de l'humidité et sous atmosphère d'azote on introduit 500 ml de dichlorométhane et 10 g de **5**. On ajoute 0,2 g de trifluorométhanesulfonate de scandium et laisse agiter 18 h à température ambiante (18 °C). On lave par une solution de $NaHCO_3$, sèche, évapore. On chromatographie sur silice en éluant par un gradient de méthanol dans le dichlorométhane. On obtient 88 % d'un produit **6.**
HPLC : ee = 94 %.
R.M.N. $^1H$ $\delta$ (ppm) : 2,70 (s,3H); 3,2-3,6 (m,2H); 4,0-4,25 (q,1H); 4,7-4,9 (m,1H); 7,28 (s,1H); 7,35-7,65 (m,6H); 7,65-785 (m,2H); 8,15-8,28 (m,2H); 8,30-8,35 (m,1H).

Produit **7** : $[(I) ; A = NH_2 , Z=CH, Z^1= CH, Z^2 = CH, X^1 = H, X^2 = H]$

1,5 g du produit **6** obtenu à l'étape précédente est hydrogéné à 80 °C dans 150 ml de méthanol, en présence de 1,3 g de ruthénium sur charbon à 5%, sous une pression de 800 kPa. Après refroidissement, filtration, et rinçage

du catalyseur par du méthanol; on évapore le solvant. Le produit est chromatographié dans un gradient de dichlorométhane enrichi en méthanol. Rdt = 55 %. CCM (DM2) :Rf = 0,25.
HPLC : ee = 94 %.
R.M.N. $^1H$ δ (ppm) :2,8 (s,3H); 3 (m,1H); 3,2 (m,1H); 3,85 (q,1H); 4,5 (q,1H); 6,4 (s,1H); 6,8 (s,1H); 7,1 (s,1H); 7,4-7,6 (m,7H); 7,7 (m,2H); 7,9 (m, 1H); 8,15 (d,1H).
I.R. : 3300, 1660, 1580, 1480, 1380,1300, 1240, 1100, 880, 780, 700 $cm^{-1}$.

EXEMPLE COMPARATIF 2

acide 2-méthyl-3-((3S) 9-amino-4-oxo-1-phényl-3,4,6,7-tétrahydro-[1,4]diazépino[6,7,1-*hi*]indol-3-yl)-4-oxo-3,4-dihydro-quinazoline-7-carboxylique méthyl ester. (**13**)

[(I); A = $NH_2$, Z = CH, $Z^1$= CH, $Z^2$ = CH, B = $CH_3$, $X^1$ =H, $X^2$ = $CO_2CH_3$ en 7.]

Ce produit est préparé à partir de la même amine nitrée **1** que pour l'Exemple 1, et de l'ester monométhytique de l'acide 2-aminotéréphtalique protégé, lui même préparé en deux étapes à partir d'acide 2-aminotéréphtalique.

1°) Synthèse de l'intermédiaire (II)

Intermédiaire **9** : acide 3-*t*-butoxycarbonylamino-*N* ((3R) 9-nitro-4-oxo-1-phényl-3,4,6,7-tétrahydro-[1,4] diazépino [6,7,1-*hi*]indol-3-yl)-téréphtalamique méthyl ester.

[ (IV) ; A = $NO_2$ , Z = CH, $Z^1$= CH, $Z^2$ = CH, $X^1$ = H, $X^2$ = $CO_2CH_3$ PG = *t*-BOC]

Dans un réacteur protégé de l'humidité on dissout sous agitation 7 g (32 mmol) d'acide 2-*t*-butoxycarbonylamino-téréphtalique 4-méthyl ester dans 250 ml de dichlorométhane sec. Le mélange est refroidi à 0 °C et on ajoute 10 g (32 mmol) de **1** dans 30 ml de dichlorométhane. On ajoute 4,32 g (32 mmol) d'HOBT et 6,6 g (32 mmol) de DCC. Après 4 h sous agitation à 0 °C, l'insoluble est filtré et le filtrat extrait successivement par une solution $HKSO_4$ 0,1 N, une solution saturée en $NaHCO_3$ et enfin par de l'eau. Le solvant est évaporé sous vide à 0 °C et le résidu purifié par chromatographie rapide sur colonne de silice en éluant par le solvant dichlorométhane /acétate d'éthyle à 5%. On obtient 10 g de **9**. Rdt = 60 %.
R.M.N. $^1H$ δ (ppm) : 1,42 (s,9H); 3,12-3,6(m,2H); 3,95-4,2(m, 1H); 4,05 (s,3H); 4,52 (m, 1H);5,62 (d, 1H); 7,4-7,65 (m, 4H); 7,65-7,72 (m, 1H); 7,72-8,1(m, 1H); 8,15 (d, 1H); 8,42 (s,1H); 8,8,82 (d, 1H) ); 10,16 (d,1H); 10,26 (s,1H); 10,88 (s,1H).
H.P.L.C. (colonne Pirkle, D-phényl-glycine, éluant hexane/éthanol 50/50, T=35°C, débit 1 ml/min, UV 254 nm) : ee = 93.2%.

Intermédiaire **10** : acide 3-amino-*N*-((3R) 9-nitro-4-oxo-1-phényl-3,4,6,7-tétrahydro-[1,4] diazépino[6,7,1-*hi*]indol-3-yl)-téréphtalamique méthyl ester.

[(III) ; A = $NO_2$, Z = CH, $Z^1$ = CH, $Z^2$ = CH, $X^1$ = H, $X^2$ = $CO_2CH_3$ en para]

Dans 1,4 1 de dichlorométhane on dissout 27 g de **9** et on ajoute lentement 140 ml d'acide trifluoroacétique. On laisse 1 h sous agitation à température ordinaire et évapore sous vide à température inférieure à 25 °C. On reprend par 1 1 de dichlorométhane et lave par une solution à 5% de $NaHCO_3$ dans de l'eau, puis avec de l'eau saturée en NaCl. On sèche sur sulfate la phase organique, et évapore à température inférieure à 25 °C. Après une chromatographie sur silice dans du dichlorométhane enrichi en méthanol, on obtient 21,4 g (95 %) d'une huile **10.**
R.M.N. $^1H$ δ (ppm) : 3,35 (s,2H, échangeables); 3,1-3,5 (m,2H); 3,9-4,15 (m,1H); 3,95 (s,3H); 4,55-4,65 (m,1H); 5,23 (s,1H); 5,60 (d,1H); 7,30-7,50 (m,6H); 7,70-7,80 (m,1H); 8,05-8,35 (m,3H).

Intermédiaire **11** : acide 3-(1=méthoxy-éthylidenamino)-*N*-((3R) 9-nitro-4-oxo-1-phényl-3,4,6,7-tétrahydro-[1,4]diazépino[6,7,1-*hi*]indol-3-yl)-téréphtalamique méthyl ester.

$$[(II) ; A = NO_2, Z = CH, Z^1 = CH, Z^2 = CH, B = CH_3 , R = CH_3, X^1 = H, X^2 = para\ CO_2CH_3]$$

On dissout 20 g de **10,** dans 750 ml de triméthyl orthoacétate. On ajoute 840 mg de trifluorométhanesulfonate de scandium, et agite à 40 °C en créant un vide de 100 à 140 hPa. On suit sur plaque de silice et en HPLC l'avancement de la réaction, il se forme l'iminoéther **11,** et du produit (I) cyclisé correspondant **12**. On évapore à siccité après 48 h. Le produit non purifié est employé dans la réaction suivante.
R.M.N. $^1H$ δ (ppm) : 1,95 (s,3H); 3,1-3,5 (m,2H); 3,88(s,3H); 3,95 (s,3H); 3,8-4,18 (m,1H); 4,5-4,8 (m,1H); 5,22 (s,1H); 5,60 (d,1H); 7,30-7,50 (m,6H); 7,70-7,80 (m,1H); 8,05-8,35 (m,3H).

2°) acide 2-méthyl-3-((3S) 9-amino-4-oxo-1-phényl-3,4,6,7-tétrahydro -[1,4]di azépino[6,7,1-*hi*]indol-3-yl)-4-oxo-3,4-dihydro-quinazoline-7-carboxylique méthyl ester. (**13**) Produit **12** : acide 2-méthyl-3-((3S) 9-nitro-4-oxo-1-phényl-3,4,6,7-tétrahydro-[1,4] diazépino[6,7,1-*hi*]indol-3-yl)-4-oxo-3,4-dihydro-quinazoline-7-carboxylique méthyl ester

$$[(I); A = NO_2\ Z = CH, Z^1 = CH, Z^2 = CH, B = CH_3, X^1 = H, X^2 = COOCH_3 \text{ en } 7]$$

Le produit **11,** qui peut contenir un peu de **12,** est dissous dans 250 ml de triméthyl orthoacétate, et on ajoute 1 g de trifluorométhanesulfonate de scandium. On porte sous agitation à température ambiante (20 °C) et ajoute 2 ml d'acide trifluoroacétique. Après 24 h, il ne resté plus de produit de départ. On évapore, reprend par du dichlorométhane, lave par une solution à 5% de $NaHCO_3$ puis par de l'eau, sèche, évapore. On chromatographie sur silice, dans un gradient de dichlorométhane enrichi en méthanol. On obtient 10,47 g de **12** sous forme de cristaux à partir du méthanol.
R.M.N. $^1H$ δ (ppm) : 2,72 (s,3H); 3,1-3,25 (m,1H); 3,35-3,48 (1H); 3,90(s,3H); 4,0-4,12 (m,1H); 4,68-4,75 (m,1H);5,20 (s,1H); 7,12 (s,1H); 7,30-7,40 (m,2H); 7,40-7,50 (m,3H); 7,97 (d,1H); 8,10-8,20 (m,2H); 8,35 (s,1H); 8,4 (s,1H).

Produit **13** :

3,0 g (5,7 mmol) de **12** sont dissous dans 150 ml de méthanol. On place la solution dans un hydrogénateur où l'on verse 3,0 g de ruthénium sur charbon à 5% et que l'on porte à une pression d'hydrogène de 800 kPa. On chauffe à 80 °C à l'intérieur du réacteur pendant 1 h. On examine sur plaque de silice après refroidissement : il n'y a plus de produit de départ. On filtre, rince, évapore et chromatographie sur silice dans du dichlorométhane enrichi en méthanol. On obtient 2 g (rdt = 71 %) de produit **13** pur.
R.M.N. $^1H$ δ (ppm) : 2,85(s,3H); 3,0-3,1 (m,1H); 3,25-3,35(m,1H); 3,6-3,85(m,2H); 3,95(s,3H); 4,6-4,7 (m,1H); 6,45 (s, 1H); 6,85(s,1H); 5,60 (d,1H); 7,15 (s,1H); 7,0-7,6 (m,5H); 8,0 (d,1H); 8,25 (d,1H); 8,45 (s,1H).
I.R.: 3400, 3330, 3220, 1770, 1650, 1620, 1590, 1560, 1480, 1440, 1380, 1300, 1240, 1160, 1090, 1010, 760, 710 $cm^{-1}$.
ee = 96 %.

3°) Produit **14**: acide (3S) 2-méthyl-3-(9-amino-4-oxo-1-phényl-3,4,6,7-tétrahydro-[1,4]diazépino[6,7,1 -*hi*]indol-3-yl)-4-oxo-3,4-dihydro-quinazoline-7-carboxylique.

$$[ (I) ; A = NH_2, Z = CH, Z^1 = CH, Z^2 = CH, B = CH_3, X^1 =H, X^2 = CO_2H \text{ en } 7]$$

Dans 20 ml de tétrahydrothiophène (THT), on verse 4g de tribromure d'aluminium et refroidit à 20 °C. Après 10 min, on ajoute 1,4 g de **13** dissous dans 10 ml de THT et laisse une nuit sous agitation à température ambiante. On ajoute de l'eau et du dichlorométhane, on sépare un solide. On évapore le filtrat. La phase aqueuse est extraite par du dichlorométhane, et évaporée. Tous les extraits sont joints et amenés à pH = 3,8 en présence d'eau et de chloroforme. On dissout dans du chloroforme contenant 15% de méthanol. On chromatographie sur silice en éluant par un gradient de méthanol dans le dichlorométhane. Les fractions contenant le produit sont jointes et chromatographiées sur phase inverse de type C18 Kromasil de 5 µm en éluant par un gradient d'acétonitrile dans de l'eau. Obtenu : 0,476 g (Rdt = 35 %) de **14,** poudre beige clair.
C.C.M. (D/MN20) : Rf= 0,37.
ee = 94 %.
R.M.N. $^1H$ δ (ppm) : 2,7(s,3H) ; 3,0-3,1 (m,1H) 3,3-3,4 (m,1H); 3,4-3,7 (m,3H); 3,8-3,95 (m,1H) 4,45-4,55 (m,1H); 6,45 (s,1H); 6,9(s,1H); 6,95 (s,1H); 7,4-7,6 (m,5H); 7,95-8,2 (m,3H).
I.R.: 3350,1680, 1480, 1380, 1300, 1240, 1170, 1110, 1010, 790, 690.

EXEMPLE 3

(3S) 3-(2-méthyl-4-oxo-4*H* pyrido[3,4-*d*]pyrimidin-3-yl)-9-amino-1-phényl-6,7-dihydro-3*H*-[1,4]diazépino[6,7,1-*hi*]indol-4-one. (**18**)

$$[(I) ; A = NH_2, Z = N, Z^1 = CH, Z^2 = CH, B = CH_3, X^1 = H, X^2 = H]$$

Cette synthèse est une illustration du procédé direct B, où l'on ne part pas d'un acide ortho aminé protégé, mais directement d'un acide ortho aminé (V').

1 °) Synthèse de l'intermédiaire (II)

Intermédiaire **15** : 2-amino-*N*-((3R) 9-nitro-4-oxo-1-phényl-3,4,6,7-tétrahydro-[1,4]diazépino[6,7,1-*hi*]indol-3-yl)-nicotinamide.

$$[(III); A = NO_2, Z = N, Z^1 = CH, Z^2 = CH, X^1 = H, X^2 = H]$$

Dans un réacteur protégé de l'humidité on dissout sous agitation 3,08 g (22,3 mmol) d'acide 2-amino-nicotinique dans 150 ml de dichlorométhane sec. On ajoute 2,51 g (18,6 mmol) d'HOBT et 3,83 g (18,6 mmol) de DCC. Le mélange est refroidi à 0 °C et on ajoute 6,0 g (18,6 mmol) de **1** dans 25 ml de dichlorométhane. Après 24 h sous agitation à 0 °C, l'insoluble est filtré et le résidu est rincé par du dichlorométhane. Les phases organiques sont évaporées et le résidu est dissout dans un excès de dichlorométhane. La phase organique est lavée par une solution $HKSO_4$ 0,1 N, une solution saturée en $NaHCO_3$ et enfin par de l'eau. Le solvant est évaporé sous vide à 0°C et le résidu purifié par chromatographie rapide sur colonne de silice en éluant par D/M1. On obtient 4,0 g d'un solide blanc cristallisé **15**. Rdt = 48 %.
C.C.M. (D/MN5) : Rf = 0,49.
R.M.N. $^1H$ δ (ppm) : 3,22-3,32 (m,1H); 3,4-3,55 (m,1H); 4,05-4,2 (m,1H); 4,7-4,8 (m,1H) 5,65 (d,1H); 6,4 (s,2H,échangeables); 6,75 (m,1H); 7,4-7,5 (m,2H); 7,5-7,6 (m,3H); 7,85 (m,1H); 7,95 (m,1H 8,25 (m,2H); 8,32(m,1H).

Intermédiaire **16**: acide *N*-[3-((3R) 9-nitro-4-oxo-1-phényl-3,4,6,7-tétrahydro-[1,4]di-azépino[6,7,1-*hi*]indol-3-ylcarbamoyl)-pyridin-2-yl]-acétimidique méthyl ester.

$$[(II);A=NO_2,Z=N,Z^1=CH,Z^2=CH,B=CH_3,X^1=H,X^2=H,R=CH_3]$$

4,0 g de **15** sont dissous dans 125 ml de triméthyl orthoacétate ; on chauffe à 40 °C sous agitation, sous un vide de 0,1 kPa pour éliminer le méthanol qui se forme dans la réaction.
Après 6 h la réaction est complète. On évapore le solvant et utilise le produit brut directement dans la réaction suivante.
R.M.N. $^1H$ δ (ppm) : 2,1 (s,3H); 3,1-3,6 (m,2H); 3,95-3,2 (m,1H); 4,1 (s,3H); 4,55-4,75 (m,1H); 5,62 (d,1H); 7,05-7,2 (m,1H); 7,25-7,55(m,7H); 8,05-8,35 (m,2H); 8,35-8,55 (m; 1H).

2°) (3S) 3-(2-méthyl-4-oxo-4*H*-pyrido[3,4-*d*]pyrimidin-3-yl)-9-amino-1-phényl-6,7-dihydro-3*H*-[1,4]diazépino(6,7,1-*hi*] indol-4-one.(**18**)

Produit 17:

On reprend **16** dans 400 ml de dichlorométhane, ajoute 240 mg (6% en poids) de trifluorométhanesulfonate de scandium et agite pendant 6 h à 40 °C. On ajoute à nouveau 500 mg de catalyseur et agite à température ordinaire pendant 6 h et évapore à siccité. On reprend le résidu par 400 ml de dichlorométhane, ajoute encore 500 mg de catalyseur.

Après 24 h, la réaction est complète. On lave par de l'eau, évapore, et obtient 5,4 g de produit brut sous forme de résine.

Produit **18** :

2,6 g (5,6 mmol) de **17** sont hydrogénés sous une pression de 800 kPa à 80 °C dans 260 ml de méthanol, en présence de 2,5 g de ruthénium sur charbon à 5% pendant 90 min. On refroidit, filtre sur un lit de silice et évapore la phase organique. On obtient 2,1 g de produit brut que l'on chromatographie sur phase inverse (colonne de C18 Kromasil 5 μm dans un gradient linéaire d'acétonitrile dans de l'eau). On obtient 300 mg de produit **18.**
ee = 94,5 %.
R.M.N. $^1H$ δ (ppm) : 2,7 (s,3H); 3,05 (m,1H); 3,3 (m,1H); 3,9 (q,1H); 4,5 (t, 1H); 5,4 (s,2H); 6,4 (s, 1H); 6,85 (s,1H); 6,9 (s,1H); 7,5 (m,5H); 7,9 (d,1H); 8,7 (d,1H); 9,1 (s,1H).

EXEMPLE 4

(3S) 3-(2-méthyl-4-oxo-4*H*-quinazolin-3yl)-9-méthyl-1-phényl-6,7-dihydro-3*H*[1,4]diazépino[6,7,1-*hi*[indol-4-one. (**23**)

$$[(I);A=CH_3,Z=CH,Z^1=CH,Z^2=CH,B=CH_3,X^1=H,X^2=H]$$

1°) Synthèse de l'intermédiaire (II)

Intermédiaire (**19**) : (3R) 3-amino-9-méthyl- 1 -phényl-6,7-dihydro-3*H*-[1,4]di-azépino[6,7,1-*hi*]indol-4-one. [(VI) ; A = $CH_3$].

Une méthode de préparation de ce produit est décrite dans la demande WO 96/11690.

Intermédiaire (**20**): acide [2-((3R) (9-méthyl-4-oxo-1-phényl-3,4,6,7-tétrahydro-[1,4]di-azépino[6,7,1-*hi*]indol-3-ylcarbamoyl)-phényl]-carbamique *t*-butyl ester.

$$[(IV) ; A = CH_3, Z = CH, Z^1= CH, Z^2 = CH, X^1 =H, X^2 = H, PG = t\text{-BOC}].$$

Dans un réacteur protégé de l'humidité on dissout sous agitation 4,07 g (17,61 mmol) de **2** dans 65 ml de dichlorométhane sec. Le mélange est refroidi à 0 °C et on ajoute 5 g (17,61 mmol) de **19** dans 65 ml de dichlorométhane. On ajoute 2,32 g (17,61 mmol) d'HOBT et 3,54 g (17,61 mmol) de DCC. Après 2 h sous agitation à 0 °C, on vérifie par C.C.M. (éluant : D/M 5) qu'il ne reste plus de **19.** Le solvant est évaporé sous vide à 20 °C et le résidu purifié par chromatographie rapide sur colonne de silice en éluant par le solvant dichlorométhane/acétone à 2%. On obtient 7,4 g d'un solide blanc cristallisé. Rdt = 82 %. C.C.M. (D/MS) : Rf = 0,76
H.P.L.C. (colonne Pirkle, D-phényl-glycine, éluant hexane/éthanol 50/50, T = 30°C ; débit : 1 ml/min, UV : 254 nm) : ee = 97 %.
$[\alpha]_D$ = + 27,5° (c = 0,0204 g/ml, acétone, Na-D 589 nm)
R.M.N. $^1H$ δ (ppm) : 1,4(s ,9H) ;2,3(s,3H) ;3,1(m,1H) ;3,35(m,1H) ; 3,9(m;1H) ; 4,5(m,1H); 5,45(d,1H); 7,0(s,1H) ; 7,10 (t,1H) ; 7,5(m,7H); 8,1(d,1H); 8,22(d,1H) ; 9,8(d,1H) ; 10,4(s,1H).

Intermédiaire **21** : 2-amino-*N*-((3R) 9-méthyl-4-oxo-1-phényl-3,4,6,7-tétrahydro-[1,4]diazépino[6,7,1-*hi*]indol-3-yl)-benzamide.

$$[(III) ; A = CH_3, Z= CH, Z^1 = CH, Z^2 = CH, X^1 = H, X^2 = H].$$

Dans un réacteur de 100 ml protégé de l'humidité et sous atmosphère d'azote, on introduit 7,4 g (145 mmol) de **20** dans 400 ml de dichlorométhane, puis, à 0 °C, 57,84 g (507,25 mmol) d'acide trifluoroacétique. On agite 1 h à 0 °C et on évapore à 20 °C sous vide. On reprend par le dichlorométhane, on lave par une solution aqueuse de $NaHCO_3$ puis par une solution de NaCl, et évapore à 20 °C. Brut : 5 g. Le résidu est purifié par chromatographie rapide sur colonne de silice en éluant par un solvant dichlorométhane/acétone à 5 %. On obtient 4,75 g d'une poudre blanche 21. Rdt = 80 %.
HPLC chirale (colonne Pirkle, D-phényl-glycine, éluant hexane/éthanol 50/50, T = 30 °C, débit : 1 ml/min, UV : 254 nm) : ee = 98 %.
C.C.M. (D/M5): Rf = 0,23.
$[\alpha]_D$ = +36,82° (c = 0,0315 g/ml, acétone, Na-D 589 nm).
R.M.N. $^1H$ $\delta$ (ppm) : 2,3(s,3H) ; 3,1(m,1H) ;3,3(m,1H) ; 3,9 (m, 1H) ; 4,5 (m,1H) ; 5,4(d,1H) ; 6,35(m,1H) ; 6,55(t,1H); 6,7(d,1H); 7,0(s,1H); 7,2(t,1H); 7,4(m,6H); 7,8(d,1H) ; 9,15(d,1H):

Intermédiaire **22** : acide *N*-[2-((3R) 9-méthyl-4-oxo-1-phényl-3,4,6,7-tétrahydro-[1,4]di-azépino[6,7,1-*hi*]indol-3-ylcarbamoyl)-phényl]-acétimidique méthyl ester

$$[ (II) ; A = CH_3, Z = CH, Z^1 = CH, Z^2 = CH, B = CH_3\ X^1 = H, X^2 = H, R = CH_3].$$

Dans un réacteur protégé de l'humidité et sous atmosphère d'azote on introduit 300 ml de triméthyl orthoacétàte et 4,5 g (11 mmol) de **21**. On place le réacteur agité sous un vide de 0,1 kPa et on chauffe à 40 °C pendant 6 h. On évapore sous vide ; la RMN confirme l'existence d'un nouveau produit **22.** Brut : 5,55 g. Le produit est employé tel quel dans l'étape suivante.
HPLC chirale (colonne Pirkle, D-phényl-glycine, éluant hexane/éthanol 50/50, T = 30 °C, débit : 1 ml/min, UV : 254 nm) : ee =99 %.
C.C.M. (D/M5) : Rf= 0,19.

2°) (3S) 3-(2-méthyl-4-oxo-4*H*-quinazolin-3yl)-9-méthyl-1-phényl-6,7-dihydro-3*H*[1,41 diazépino[6,7,1-*hi*]indol-4-one. (**23**)

Dans un réacteur protégé de l'humidité et sous atmosphère d'azote, on introduit 300 ml de dichlorométhane et 5,55 g de **22.** On ajoute 0,117 g de trifluorométhanesulfonate de scandium, et laisse agiter 28 h à température ambiante (18 °C). On lave par une solution de $NaHCO_3$ sèche, évapore. On chromatographie sur silice en éluant par un gradient d'acétone dans le dichlorométhane. On obtient 0,65 g du produit **23.**
HPLC chirale (colonne Pirkle, D-phényl-glycine, éluant : hexane/éthanol 50/50, T = 30 °C, débit 1 ml/min, UV : 254 nm) : ee = 99 %.
C.C.M. (D/M5) : Rf = 0,23.
R.M.N. $^1H$ $\delta$ (ppm) : 2,35 (s,3H) ; 3,1 (m,1H); 3,3 (m,1H) ; 3,95 (m,1H) ; 4,7 (m,1H) ; 7,0 - 7,80 (m,12H) ; 8,20 (s,1H).

EXEMPLE 5

(3S) 9-méthoxy-3-(2-méthyl-4-oxo-4*H*-quinazolin-3-yl)-1-phényl-6,7-dihydro-3*H*[1,4]diazépino[6,7,1-*hi*]indol-4-one. **(28)**

$$[(1); A = OCH_3, Z = CH, Z^1 = CH, Z^2 = CH, B = CH_3, X^1 = H, X^2 = H]$$

1°) Synthèse de l'intermédiaire (II)

Intermédiaire **24** : (3R) 3-amino-9-méthoxy-1-phényl-6,7-dihydro-3*H* [1,4]diazépino [6,7,1-*hi*]indol-4-one. [(VI) ; A = $OCH_3$].

7,1 g (231 mmol) de (3R,S) 3-amino-9-méthoxy-1-phényl-6,7-dihydro-3*H*=[1,4]di-azépino[6,7,1-*hi*]indol-4-one , préparée tel qu'il est enseigné dans la demande de brevet WO 96/11690, sont portés au reflux dans 17,75 ml d'acétonitrile ; on y ajoute 8,92 g (23,1 mmol) d'acide di *p*-toluoyl-D-tartrique préalablement porté au reflux dans 17,75 ml d'acétonitrile. L'addition sous agitation se fait très rapidement. On maintient le reflux 5 min avant de laisser reposer 18 h. Filtrer le précipité, le rincer avec 35 ml d'acétonitrile, le sécher sous 0,1 kPa à 35 °C. On obtient 8,5 g de sel qu'on reprend dans 85 ml. d'acétonitrile ; on porte au reflux 5 min et on laisse reposer au moins 4 h. Filtrer le précipité , le rincer avec 40 ml d'acétonitrile, sécher sous 0,1 kPa à 35 °C. On obtient 5 g de sel. On libère la base, en reprenant les 5g de sel dans 100 ml d'une solution molaire de soude préalablement refroidie à 0 °C, puis on extrait avec 3 fois 200 ml d'acétate d'isopropyle. Laver la phase organique avec une solution saturée de NaCl, sécher sur sulfate de sodium, et évaporer. On obtient 2,2 g d'intermédiaire **24.**
HPLC chirale (colonne Pirkle, D-phényl-glycine, éluant : hexane/ isopropanol 50/50, T = 20 °C, débit : 1,2 ml/min, UV : 254 nm) : ee = 99 %.
Préparation de l'échantillon pour l'HPLC chirale : le produit doit être dérivatisé avec 2 équivalents de p-tolyl-isocyanate dans le dichlorométhane, puis dilué dans l'isopropanol à 0,5 mg/ml avant d'être injecté.

Intermédiaire **25** : acide [2-((3R) (9-méthoxy-4-oxo-1-phényl-3,4,6,7-tétrahydro-[1,4]di-azépino[6,7,1-*hi*]indol-3-ylcarbamoyl)-phényl]-carbamique *t*-butyl ester.

[(IV) ; A = $OCH_3$ , Z = CH, $Z^1$ = CH, $Z^2$ = CH, $X^1$ =H, $X^2$ = H, PG = *t*-BOC].

Dans un réacteur protégé de l'humidité on dissout sous agitation 1,7 g (7,16 mmol) de **2** dans 55 ml de dichlorométhane sec. Le mélange est refroidi à 0 °C et on ajoute 2,2 g (7,16 mmol) de **24** dans 55 ml de dichlorométhane. On ajoute 0,97 g (7,16 mmol) d'HOBT et 1,47 g (7,16 mmol) de DCC. Après 8 h sous agitation à 0 °C puis la nuit à température ambiante, on vérifie par C.C.M. (éluant : D/M 5) qu'il ne reste plus de **24.** Le solvant est évaporé sous vide à 30 °C et le résidu purifié par chromatographie rapide sur colonne de silice en éluant par le solvant dichlorométhane /acétone à 3%. On obtient 3,2 g d'un solide blanc cristallisé **25.** Rdt = 85 %
C.C.M. (D/M5): Rf = 0,72.
H.P.L.C. (colonne Pirkle, D-phényl-glycine, éluant hexane/éthanol 50/50, T = 30 °C ; débit : 1 ml/min, UV : 254 nm) : ee = 98 %.
$[\alpha]_D$ = + 11 ° (c = 0,04507 g/ml, acétone, Na-D 589 nm)
R.M.N. $^1H$ $\delta$ (ppm) : 1,45 (s ,9H) ; 3,1 (m,1H); 3,4 (m,1H); 3,7 (s,3H) ; 3,9 (m,1H); 4,5 (m,1H) ; 5,5 (d,1H) ; 6,65 (s,1H) ; 7,10 (t,1H) ; 7,3 (s,1H) ; 7,5 (m,6H) ; 8,25 (d,1H) ; 9,8 (d,1H) ; 10,4 (s,1H).

Intermédiaire **26:** 2-amino-*N*-((3R) 9-méthoxy-4-oxo-1-phényl-3,4,6,7-tétrahydro-[1,4] diazépino[6,7,1-*hi*]indol-3-yl)-benzamide.

[(III) ; A = $OCH_3$ , Z = CH, $Z^1$ = CH, $Z^2$ = CH, $X^1$ =H, $X^2$ = H].

Dans un réacteur de 500 ml protégé de l'humidité et sous atmosphère d'azote, on introduit 3,1 g (5,88 mmol) de **25** dans 260 ml de dichlorométhane, puis, à 0 °C, 23,53 g (20,604 mmol) d'acide trifluoroacétique. On agite 1 h à 0 °C et on évapore à 20 °C sous vide. On reprend par le dichlorométhane, lave par une solution de $NaHCO_3$ puis par une solution de NaCl, et évapore à 20 °C. On obtient 2,75 g de **26** pur.
Le produit est employé tel quel dans l'étape suivante.
HPLC chirale (colonne Pirkle, D-phényl-glycine, éluant hexane/éthanol 50/50, T = 30 °C, débit : 1 ml/min, UV : 254 nm) : ee = 99 %.
C.C.M. (D/M5) : Rf = 0,20.
$[\alpha]_D$ = +20,5° (c = 0,02923 g/ml, acétone, Na-D 589 nm).

R.M.N. $^1H$ δ (ppm) : 3,15 (m,1H) ; 3,45 (m,1H) ; 3,7 (s,3H) ; 3,9 (m,1H) ; 4,5 (m,1H) ; 5,45 (d,1H) ; 6,4 (m,1H) ; 6,55 (t,1H); 6,65 (s,1H); 6,7 (d,1H) ; 7,3 (s,1H) ; 7,55 (m,5H); 7,8(d,1H) ; 9,10 (d,1H).

Intermédiaire **27 :** acide *N*-[2-((3R) 9-méthoxy-4-oxo-1-phényl-3,4,6,7-tétrahydro-[1,4]di-azépino[6,7,1-*hi*]indol-3-ylcarbamoyl)-phényl ]-acétimidique méthyl ester

$$[(II) ; A= OCH_3 , Z = CH, Z^1 =CH, Z^2=CH, B = CH_3 , X^1 =H, X^2 = H, R= CH_3].$$

Dans un réacteur protégé de l'humidité et sous atmosphère d'azote, on introduit 200 ml de triméthyl orthoacétate et 2,5 g (5,86 mmol) de **26.** On place le réacteur agité sous un vide de 0,1 kPa et on chauffe à 40 °C pendant 6 h. On évapore sous vide. La RMN confirme l'existence du nouveau produit **27,** et la présence de traces du produit de départ **26.**
Brut : 2,5 g. Le produit est employé tel quel dans l'étape suivante.
HPLC chirale (colonne Pirkle, D-phényl-glycine, éluant hexane/éthanol 50/50, T=30°C, débit 1 ml/min, UV : 254 nm) : ee = 99 %.
C.C.M. (D/M5) : Rf= 0,22.

2°) (3S) 9-méthoxy-3-(2-méthyl-4-oxo-4*H*-quinazolin-3-yl)-1-phényl-6,7 -dihydro-3*H*[1,]1diazépino[6,7,1-*hi*]indol-4-one. (**28**)

Dans un réacteur protégé de l'humidité et sous atmosphère d'azote, on introduit 200 ml de dichlorométhane et 2,5 g de **27.** On ajoute 0,051 g de trifluorométhanesulfonate de scandium et laisse agiter 36 h à température ambiante (18 °C). On lave par une solution de $NaHCO_3$, sèche, évapore. On chromatographie sur silice en éluant par un gradient d'acétone dans le dichlorométhane. On obtient 1,7 g d'un produit **28** pur.
HPLC chirale (colonne Pirkle, D-phényl-glycine, éluant : hexane/éthanol 50/50, T = 30 °C, débit : 1 ml/min, UV : 254 nm) : ee = 99 %.
C.C.M. (D/M5) : Rf = 0,20.
R.M.N. $^1H$ δ (ppm) : 2,8 (s,3H) ; 3,1 (m,1H) ; 3,4 (m,1H) ; 3,75 (m,1H) ; 3,9 (m,1H) ; 4,7 (d,1H) ; 6,70 (m,1H) ; 7,10 (s, 1H) ; 7,2 (s,1H) ; 7,3 - 7,85 (m,8H) ; 8,2 (d,1H).

EXEMPLE 6

Synthèse du composé **12** [(I); A = $NO_2$, Z = CH, $Z^1$ = CH, $Z^2$ = CH, B = $CH_3$, $X^1$ = H, $X^2$ = $COOCH_3$ en 71 à partir de l'intermédiaire 10 [ (III); A = $NO_2$, Z = CH, $Z^1$ = CH, $Z^2$ = CH, $X^1$ = H, $X^2$ = $CO_2CH_3$ en para] en présence de différents catalyseurs.

2,8g de produit **10** sont dissous dans 50 ml de triméthyl orthoacétate et sont laissés 24 heures à température ambiante. La chromatographie sur couche mince permet de vérifier qu'il reste peu de produit de départ. On observe aussi la formation d'imines en faible quantité. 100mg de catalyseur sont ensuite ajoutés en une seule fois à 2ml de solution (ce qui correspond à 112mg de produit de départ). Cette réaction est effectuée avec 6 catalyseurs différents. Une réaction sans catalyseur est également réalisée. L'évolution de la réaction est suivie par chromatographie sur couche mince (éluant D/M1,5).
Au bout de 48 heures, chaque milieu réactionnel est repris dans 100ml de chlorure de méthylène. Après ajout de HCl 1N et dissolution du catalyseur, les opérations suivantes sont réalisées : décantation, lavage au $NaHCO_3$, séchage, évaporation. On pèse finalement le produit obtenu.
Des solutions à 1 mg/ml des différents produits sont préparées puis les produits sont analysés par HPLC analytique à l'aide des méthodes suivantes:

1) colonne de type C18 Kromasil de 5 µm (250*4,6mm) en éluant par un gradient d'acétonitrile dans de l'eau.

$t_{0min}$ : 20% acétonitrile,
$t_{25min}$ : 95% acétonitrile).

2) colonne de type Pirckle D-phénylglycine (250*4,6mm) en éluant par un mélange de hexane/éthanol (50/50)

Les temps de rétention pour les deux isomères optiques sont de 10,15min pour l'énantiomère R et 14,60min pour l'énantiomère S.

**Résultats :**

| Catalyseur utilisé | Masse de produit obtenue | % de produit cyclisé (**12**) | Pureté optique | Produits secondaires | Produit non cyclisé |
|---|---|---|---|---|---|
| Témoin sans catalyseur | 106mg | 0% | >98% | | 99% |
| $Sc(OSO_2CF_3)_3$ | 106mg | 92% | >98% | 5% | 0% |
| *$AlBr_3$** | *98mg* | *10%* | *épimérisation totale* | *83%* | *0%* |
| *$AlCl_3$** | *110mg* | *44%* | *>98%* | *30%* | *0%* |
| *$BF_3$** | *130mg* | *26%* | *>98%* | *64%* | *1%* |
| $ZnCl_2$ | 120mg | 76% | >98% | 20% | 0% |
| *$CF_3COOH$** | *105mg* | *22%* | *>98%* | *47%* | *28%* |

** essais comparatifs*

**Revendications**

**1.** Procédé de préparation des *enantiomères des* diazépino-indolones de formule

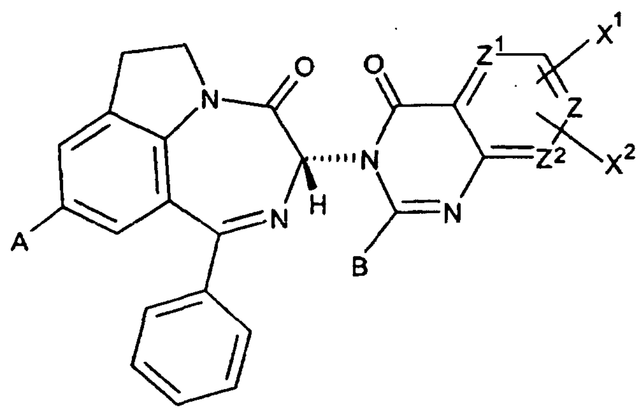

**(I)**

dans laquelle :

- A est hydrogène, $C_1$-$C_4$ alkyle, hydroxy, $C_1$-$C_4$ alcoxy, nitro, cyano, ou $NR^1R^2$; $R^1$ et $R^2$ sont indépendamment hydrogène, $C_1$-$C_4$ alkyle, ou forment ensemble avec l'atome d'azote auquel ils sont liés un cycle ayant 4 ou 5 atomes de carbone ;
- B est hydrogène ou $C_1$-$C_4$ alkyle;
- Z est CH, alors $Z^1$ et $Z^2$ sont ensemble CH ou N ; ou Z est N, alors $Z^1$ et $Z^2$ sont CH;
- $X^1$ et $X^2$ sont indépendamment hydrogène, $C_1$-$C_4$ alkyle, $-(CH_2)_j-OR^3$, halogène, cyano,
- O-$C_1$-$C_6$ alkyle, $-C(=O)R^4$, $-C(=O)OR^5$, $-C(=O)NR^6R^7$, ou

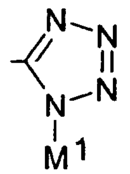 , ou 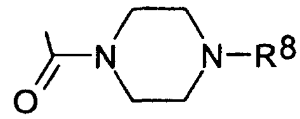 ;

$R^3$, $R^4$, et $R^5$ sont indépendamment hydrogène, $C_1$-$C_6$ alkyle, benzyle, phénéthyle, ou - $Q^1$-$Q^2$;

$R^6$ est hydrogène, ou $C_1$-$C_4$ alkyle;

$R^7$ est hydrogène , $C_1$-$C_4$ alkyle, $-CHR^A-C(=O)OM^2$, ou $-Q^3-Q^4$ ;

$R^8$ est hydrogène, $C_1$-$C_4$ alkyle, ou $-Q^5$ $-Q^6$ ;

$R^A$ est un résidu d'α-amino-acide naturel, l'atome de carbone auquel il est lié pouvant avoir soit la configuration S, soit la configuration R ;

$Q^1$ est $-(CH_2)_k-(CHOH)_m-(CH_2)_p-$ ;

$Q^2$ est hydroxy, -O- $C_1$-$C_6$ alkyle, -OC(=O)- $C_1$-$C_6$ alkyle, ou 4-morpholinyle ;

$Q^3$ et $Q^5$ sont indépendamment une liaison, $-CH_2$ -, $-(CH_2)_2$ -, ou $-(CH_2)_3$ - ;

$Q^4$ est $-NM^3M^4$, ou 4-morpholinyle ;

$Q^6$ est $-M^5$ ou $-OM^6$ ;

$M^1$, $M^2$, $M^3$, $M^4$, $M^5$, et $M^6$ sont indépendamment hydrogène ou $C_1$-$C_4$ alkyle ;

j est 1,2, ou 3 ; k est 1,2, ou 3 ;

m est 0, 1, 2, 3, ou 4 ; p est 0, 1, 2, ou 3, sous réserve que si m > 0, alors p > 0 ; leurs sels; *et* solvates, qui comprend la cyclisation intramoléculaire à une température qui ne dépasse pas 40 °C, d'un produit de formule

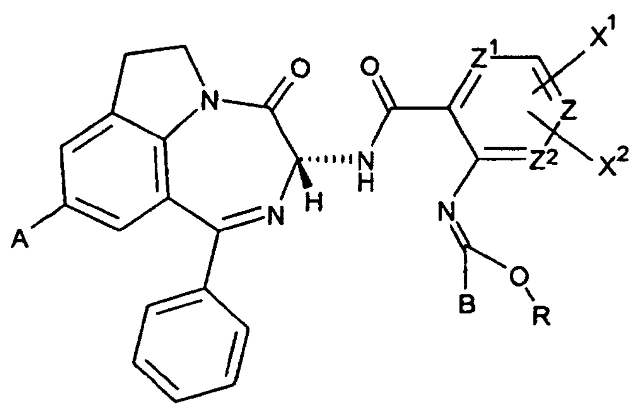

,

dans laquelle, A, Z, $Z^1$, $Z^2$, B, $X^1$ et $X^2$ ont la signification définie ci-dessus, et R est $C_1$-$C_4$ alkyle, en présence d'un catalyseur acide de Lewis faible qui est le scandium trifluorométhanesulfonate; l'aluminium trifluorométhanesulfonate; les trifluorométhanesulfonates de lanthanide, de silicium, de magnésium, d'étain (II), de cuivre (II), de zinc ou d'argent ; le diméthoxy- dicyclopentadiényl-titane (IV) ; le bis (trifluorométhanesulfonate)dicyclopentadiényl-titane (IV) ; l'acétylacétonate de fer (III), d'aluminium ou de zinc ; le diacétate de zinc ; le diméthoxy magnésium ; le triisopropoxy aluminium ; le tétrabutoxy titane (IV) ; le tétraisopropoxy titane (IV); le triméthyl bore ; le triéthyl

aluminium ; le diéthyl zinc ; le triisobutyl aluminium ; le tétrabutyl étain (IV) ; le triphényl bore ; le triphényl antimoine ; ou les halogénures de zinc, d'étain (II), d'antimoine, de titane, de scandium, d'indium, de gallium, ou de mercure (II).

**2.** Procédé selon la revendication 1 dans lequel ledit acide de Lewis faible est le scandium trifluorométhanesulfonate.

**3.** Procédé selon la revendication 1 **caractérisé en ce qu'**il comprend une étape additionnelle où un produit (I) dans lequel A est $-NO_2$ est réduit en un produit (I) dans lequel A est $-NH_2$.

**4.** Procédé selon la revendication 1 dans lequel A est $-NH_2$ , B est $CH_3$ , et $X^1$ et $X^2$ sont indépendamment hydrogène, halogène ou $-C(=O)OR^5$.

**Patentansprüche**

**1.** Verfahren zur Herstellung von Enantiomeren der Diazepino-Indolone mit der Formel:

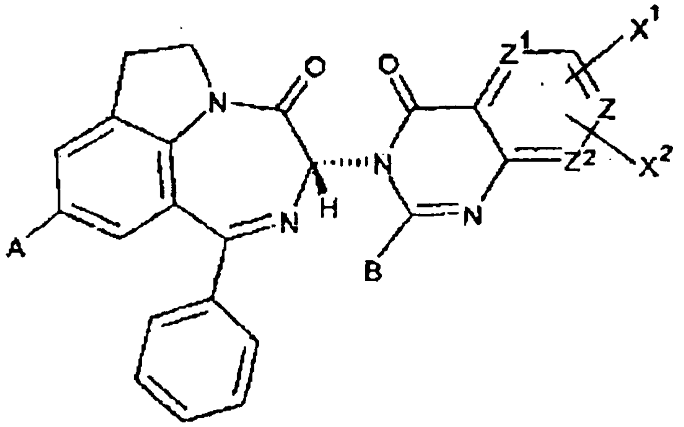

(I)

wobei:

- A Wasserstoff, $C_1$-$C_{4-}$ Alkyl, Hydroxy, $C_1$-$C_{4-}$ Alkoxy, Nitro, Cyan oder $NR^1R^2$ ist;
  $R^1$ und $R^2$ sind jeweils unabhängig Wasserstoff, $C_1$-$C_4$ Alkyl oder bilden zusammen mit dem Stickstoffatom, an das sie gebunden sind, einen Zyklus, der 4 oder 5 Kohlenstoffatome enthält;
- B Wasserstoff oder $C_1$-$C_4$ Alkyl ist,
- Z CH ist - in diesem Fall sind $Z^1$ und $Z^2$ zusammen CH oder N - oder Z N ist - dann sind $Z^1$ und $Z^2$ CH -,
- $X^1$ und $X^2$ jeweils unabhängig Wasserstoff, $C_1$-$C_4$ Alkyl, $-(CH_2)_j-OR^3$, ein Halogen, Cyan, $-O-C_1-C_6$Alkyl, $-C(=O)R^4$, $-C(=O)OR^5$, $-C(=O)NR^6R^7$ oder

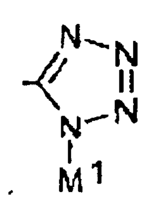

, oder

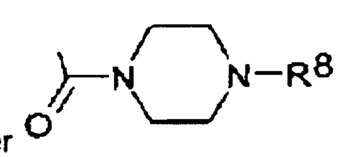

sind,

$R^3$, $R^4$ und $R^5$ sind jeweils unabhängig Wasserstoff, $C_1$-$C_6$ Alkyl,Benzyl, Phenethyl oder $-Q^1-Q^2$,

$R^6$ ist Wasserstoff oder $C_1$-$C_4$Alkyl,
$R^7$ ist Wasserstoff, $C_1$ -$C_4$ Alkyl, -$CHR^A$ -C(=O)$OM^2$ oder -$Q^3$-$Q^4$,
$R^8$ ist Wasserstoff, $C_1$ -$C_4$ Alkyl oder -$Q^5$-$Q^6$,
$R^A$ ist ein natürlicher α-Aminosäurerest, wobei das Kohlenstoff-atom, an das er gebunden ist, entweder die Konfiguration S oder Konfiguration R haben kann,
$Q^1$ ist -$(CH_2)_k$-$(CHOH)_m$-$(CH_2)_p$-,
$Q^2$ ist Hydroxy, -O- $C_1$-$C_6$ Alkyl, -OC(=O)- $C_1$-$C_6$ Alkyl oder 4-Morpholinyl,
$Q^3$ und $Q^5$ sind unabhängig eine Verbindung, -$CH_2$-, -$(CH_2)_2$- oder -$(CH_2)_3$-,
$Q^4$ ist -$NM^3M^4$ oder 4-Morpholinyl,
$Q^6$ ist -$M^5$ oder -$OM^6$,
$M^1$, $M^2$, $M^3$, $M^4$, $M^5$ und $M^6$ sind unabhängig Wasserstoff oder $C_1$-$C_4$ Alkyl,
j ist 1, 2 oder 3 und k ist 1, 2 oder 3,

m 0, 1, 2, 3 oder 4, p 0, 1, 2 oder 3 ist, mit dem Vorbehalt, dass wenn $m > 0$, $p > 0$ ist; sowie zur Herstellung deren Salze und Solvate,
welches die intramolekulare Zyklisierung bei einer Temperatur, die 40°C nicht übersteigt, eines Produktes mit der Formel

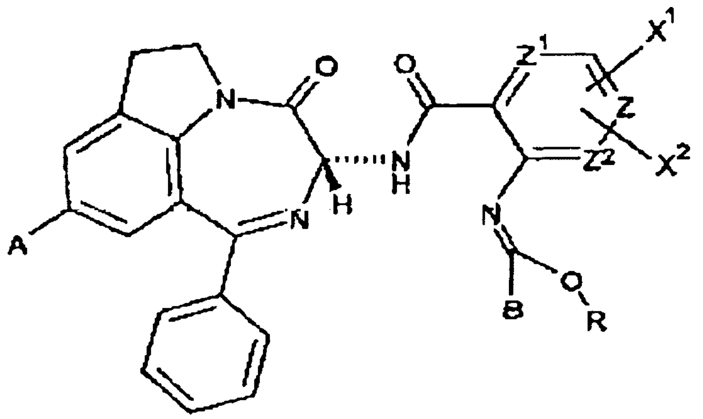

umfasst, wobei
A, Z, $Z^1$, $Z^2$, B, $X^1$ und $X^2$ die oben definierte Bedeutung haben und R $C_1$-$C_4$ Alkyl ist in Anwesenheit eines schwachen Lewis-Säure-Katalysators, der sein kann: das Scandium-Trifluormethansulfonat, das Aluminium-Trifluormethansulfonat, die Lanthanid-, Silizium-, Magnesium-, Zinn(II)-, Kupfer(II)-, Zink- oder Silber-Trifluormethansulfonate, das Dimethoxy-Dicyclopentadienyl-Titan(IV), das Bis(Trifluormethansulfonat) Dicyclopentadienyl-Titan(IV), das Eisen(III)-, Aluminium- oder Zink-Acetylacetonat, Zink-Diacetat, Dimethoxy-Magnesium, Triisopropoxy-Aluminium, Tetrabutoxy-Titan(IV), Tetraisopropoxy-Titan(IV), Trimethyl-Bor, Triethyl-Aluminium, Diethyl-Zink, Triisobutyl-Aluminium, Tetrabutyl-Zinn(IV), Triphenyl-Bor, Triphenyl-Antimon oder die Zink-, Zinn(II)-, Antimon-, Titan-, Scandium-, Indium-, Gallium- oder Quecksilber(II)- Halogenide.

**2.** Verfahren nach Anspruch 1, wobei die schwache Lewis-Säure das Scandium-Trifluormethansulfonat ist.

**3.** Verfahren nach Anspruch 1 **dadurch gekennzeichnet, dass** es einen zusätzlichen Verfahrensschritt umfasst, bei welchem ein Produkt (I), in dem A - $NO_2$ ist, zu einem Produkt (I), in dem A -$NH_2$ ist, reduziert wird.

**4.** Verfahren nach Anspruch 1, wobei A -$NH_2$, B $CH_3$ ist und $X^1$ und $X^2$ unabhängig Wasserstoff, ein Halogen oder -C(=O)$OR^5$ sind.

## Claims

**1.** A method for preparing enantiomers of diazepinoindolones of formula

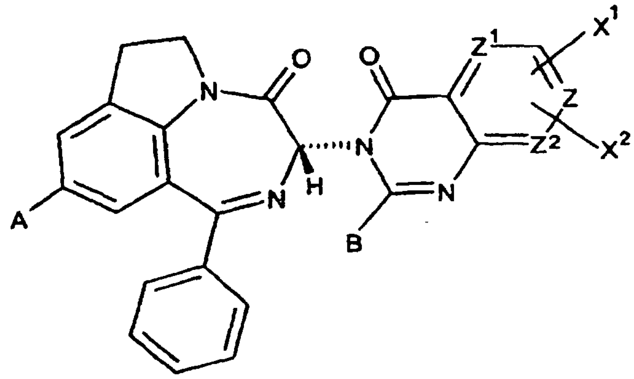

(I)

wherein:

- A is hydrogen, $C_1$-$C_4$ alkyl, hydroxyl, $C_1$-$C_4$ alcoxy, nitro, cyano, or $NR^1R^2$;
  $R^1$ and $R^2$ independently are hydrogen, $C_1$-$C_4$ alkyl, or together form with the nitrogen atom to which they are attached, a ring with four or five carbon atoms;
- B is hydrogen or $C_1$-$C_4$ alkyl;
- Z is CH, then $Z^1$ and $Z^2$ together are CH or N; or Z is N, $Z^1$ and $Z^2$ then are CH;
- $X^1$ and $X^2$ independently are hydrogen, $C_1$-$C_4$ alkyl,
- $(CH_2)_j$-$OR^3$, halogen, cyano, -O-($C_1$-$C_6$ alkyl), -C(=O)$R^4$,
- C(=O)$OR^5$, -C(=O)$NR^6R^7$, or

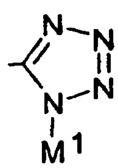

, or

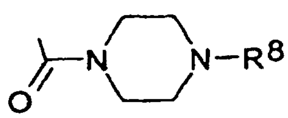

;

$R^3$, $R^4$, and $R^5$ independently are hydrogen, $C_1$-$C_6$ alkyl, benzyl, phenethyl, or -$Q^1$-$Q^2$;
$R^6$ is hydrogen or $C_1$-$C_4$ alkyl;
$R^7$ is hydrogen, $C_1$-$C_4$ alkyl, $CHR^A$-C(=O)$OM^2$ or -$Q^3$-$Q^4$;
$R^8$ is hydrogen, $C_1$-$C_4$ alkyl, or -$Q^5$-$Q^6$;
$R^A$ is a natural α-amino-acid radical, the carbon atom to which it is attached may either have the S configuration or the R configuration;
$Q^1$ is - $CH_2)_k$-$(CHOH)_m$-$(CH_2)_p$-;
$Q^2$ is hydroxy, -O-($C_1$-$C_6$ alkyl),
- OC(=O)-($C_1$-$C_6$ alkyl), or 4-morpholinyl;
$Q^3$ and $Q^5$ independently are a bond, -$CH_2$-, -$(CH_2)_2$-, or -$(CH_2)_3$-;
$Q^4$ is -$NM^3M^4$, or 4-morpholinyl;
$Q^6$ is -$M^5$ or -$OM^6$;
$M^1$, $M^2$, $M^3$, $M^4$, $M^5$, $M^6$ independently are hydrogen or $C_1$-$C_4$ alkyl;
j is 1, 2, or 3; k is 1, 2 or 3;

m is 0, 1, 2, 3 or 4; p is 0, 1, 2 or 3, with the proviso that if m > 0, then p > 0;
salts and solvates thereof,
which comprises the intramolecular cyclization at a temperature not exceeding 40°C, of a product of formula

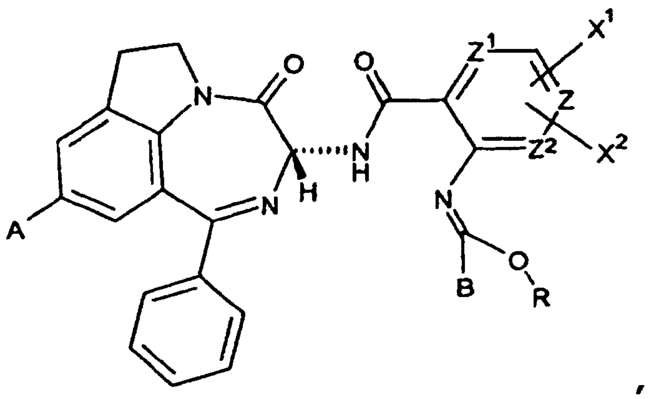

wherein, A, Z, $Z^1$, $Z^2$, B, $X^1$ and $X^2$ have the meaning defined above, and R is $C_1$-$C_4$ alkyl, in the presence of a weak Lewis acid catalyst which is scandium trifluoromethanesulfonate; aluminium trifluoromethanesulfonate; lanthanide, silicon, magnesium, tin(II), copper(II), zinc or silver trifluoromethanesulfonates; dimethoxy-dicyclopentadienyl-titanium(IV); bis(trifluoromethane-sulfonate)dicyclopentadienyl-titanium(IV); iron(III), aluminium or zinc acetylacetonate; zinc diacetate; dimethoxy magnesium; triisopropoxy aluminium; tetrabutoxy titanium(IV); tetraisopropoxy titanium(IV); trimethyl boron; triethyl aluminium; diethyl zinc; triisobutyl aluminium; tetrabutyl tin(IV); triphenyl boron; triphenyl antimony; or zinc, tin(II), antimony, titanium, scandium, indium, gallium, or mercury(II) halogenides.

2. The method according to claim 1, wherein said weak Lewis acid is scandium trifluoromethanesulfonate.

3. The method according to claim 1, **characterized in that** it comprises an additional step in which a product (I) wherein A is $-NO_2$, is reduced into a product (I) wherein A is $-NH_2$.

4. The method according to claim 1, wherein A is $-NH_2$, B is $CH_3$ and $X^1$ and $X^2$ independently are hydrogen, halogen or $-C(=O)OR^5$.